# EUROPEAN PATENT APPLICATION

(11) **EP 1 911 469 A2**
(43) Date of publication of application: **16.04.2008**
(21) Application number: 07252721.1
(22) Date of filing: 06.07.2007
(51) Int. Cl.: A61L 27/06, A61L 27/32, A61L 27/04, A61L 31/02, A61L 31/08

(54) **A titanium implant and a process for the preparation thereof**

(30) Priority: 10.10.2006 NO 20064595
(71) Applicant: Rölla, Gunnar, 0265 Oslo (NO)
(72) Inventor: Rölla, Gunnar, 0265 Oslo (NO)
(74) Representative: Golding, Louise Ann

(57) **Abstract**

The invention provides a titanium implant having increased retention after insertion in bone. The implant comprises phosphate groups bound to the metallic titanium. Also provided is a process for preparing said titanium implant, wherein the oxide layer formed on the surface of a conventional titanium implant is removed to expose the metallic titanium. The exposed implant is then treated with a phosphate solution in the absence of oxygen to bond phosphate groups to the titanium surface, said groups securing a firm attachment of the implant to the bone into which it is inserted.

## Description

Titanium or titanium alloys are frequently used in surgical implants, particularly in relation to bone surgery. Such implants are biocompatible and as strong as steel. However, if exposed to atmospheric oxygen, machined surfaces of titanium are covered by an oxide layer which shields the properties of the metal, thus presenting an inert surface.

Titanium implants are widely used in loadbearing applications such as hip prostheses and dental implants, due to their biocompatibility and mechanical strength. Direct bonding between bone and the metal is rarely seen, due to the inert layer of oxide which covers the surfaces of such implants. It is interesting to note that whereas most authors consider the oxide layer as being the basis for the biocompatibility of titanium implants, a small group believes that the properties of the oxide-covered titanium can be improved. One approach is applying bioactive materials on titanium implants to cover the oxide layer. Application of bioactive ceramics like hydroxyapatite which is a bone analogue, is believed to improve the biological properties of implants. Conventional thermal plasma spraying has been used to apply hydroxyapatite or calcium phosphate on titanium oxide, but said process has been unable to provide the desired product. Various low temperature thin film techniques have been developed recently to extend the clinical use of ceramic-coated implants. A clinical problem has been that such implants cannot be immobilized by being cemented to bone, which is the traditional method. Another problem is the low mechanical strength of ceramics.

Increased retention of implants in bone can be obtained by mechanical or chemical procedures which increase their surface area and thereby improve osseo-integration and strength of attachment. Grit blasting of implant surfaces with abrasive particles is also a well established method to obtain this effect. Such implants are usually covered by oxide. Treatment of implants with high concentrations of hydrofluoric acid has been used to obtain an extensively etched surface where pores and undercuts provide mechanical retention, as this acid is able to erode the titanium surface.

WO 95/17217 and US 2005/0161440 (Ellingsen et al.) describe a brief treatment of titanium implants with dilute hydrofluoric acid, which are shown to provide an improved contact between implant and bone, as demonstrated in animal experiments. The beneficial effect is thought to be due, at least in part, to fluorine or fluoride being retained on the titanium implant. The authors state that it is not possible to define the characteristics of the fluorine-treated surface, except that the desired characteristic of the surface is provided by the said effect.

In Documentation on Astra Tech implants, Issue 3 (2005) there is a reference to calcium phosphate precipitation in seeding experiments on implants, and speculations are made concerning possible similar reactions in vivo. These speculations thus relate to implants where precipitations occur in vitro, prior to its introduction in bone. A stimulation of osteoprogenitor cells with a subsequent elevation of alkaline phosphatase is postulated. The formation of calcium phosphate on implants (before clinical use) is also suggested to foster covalent bonds between the implant and bone. The pretreatment of the implant with calcium phosphate is speculative, and no data showing that such treatments would enhance attachment between bone and implant are available. Ample time for such experiments to be performed has been available. The fact that Hepes buffer is used in the calcium fluoride solution, and that precipitation of visible deposition of calcium phosphate occurs, represent difficult problems (Hepes buffer causes faster blood clotting which makes it unsuitable for clinical use).

The present invention aims at improving the attachment of titanium implants in bone. As the inert oxide layer which usually covers implants is removed, the titanium metal as such is exposed. As titanium is a transition metal and exhibits empty valence shell orbitals, it has high affinity for a number of ligands which can provide electrons and thereby form coordinate complexes with the titanium metal. The ranking of the strength of relevant ligands is as follows:

chloride<fluoride<hydroxyl<phosphate.

It can be seen that the oxygen-containing ligands have higher affinity for titanium than the halogens. Phosphate has a particularly high affinity for titanium as it is polydentate and provides 2-4 oxygen moieties, which can all form coordinate complexes with the transition metal. The exposed transition metal thus has other properties than oxide covered titanium, which is usually described as inert.

The present invention provides a titanium implant having increased retention after insertion in bone, wherein the implant comprises phosphate groups complex bound to the titanium surface, said groups securing a firm attachment of the implant to bone into which it is inserted.

Another aspect of the invention is a process for preparing a titanium implant having increased retention after insertion in bone, by preparing an implant in a per se known manner and then removing the oxide layer formed on the surface of the implant to expose the metallic titanium, the exposed implant is then treated with a phosphate solution in the absence of oxygen whereby phosphate groups will be complex bound to the titanium surface, said groups securing a firm attachment of the implant to bone into which it is inserted.

The present invention contains a description of a convenient and inexpensive method by which the oxide layer which covers the surfaces of titanium implants is replaced by phosphate groups, which are covalently bonded to the titanium implant by coordinate complexes. These firmly bound phosphate groups will attract calcium from the tissue fluid when the implant is placed in bone, and initiate a bone formation where the bone is firmly attached to the implant surface.

The oxide layer can be removed by mechanical or chemical procedures. It is preferred that these procedures are performed under argon to avoid reoxidation of the implant.

Polishing of the titanium surface with inert abrasives like diamond powder or oxide free titanium are examples of suitable mechanical procedures. The implants are then transferred to phosphate solutions to obtain the phosphate bonding to the transition metal surfaces described above. The ligand (phosphate) provides electrons for the empty valence shells of titanium according to G.N. Lewis.

Chemical removal of the oxide layer can be obtained by exposure of the titanium implants to halogen acids whereby titanium halides are formed, replacing the oxide as known per se. Dilute hydrofluoric acid is known to be more effective than the acids of the other halogens. After this procedure the titanium halides are transferred to a phosphate solution, whereby the oxygen moieties of the phosphate group displace the halogen, as the former is the stronger ligand.

Both the mechanical and the chemical treatment provide titanium with a surface layer of phosphate, where the phosphate groups are complex bonded to the titanium surfaces, as discussed above. Such a surface will attract calcium ions when exposed to calcium ions in the tissue fluids when used clinically, and will thus initiate a bone formation firmly attached to the implant.

Titanium/phosphate complexes are well known compounds which are used in industrial scale ion exchange procedures to remove heavy metals from hot wastewater, where organic ion exchange material are untenable. The titanium used for this purpose is produced by the FFC Cambridge method and is in the form of a sponge with a very high surface area. This compound is also used for removal of radioactivity, for renal dialysis and for removal of calcium from wine. It is a stable compound which is not decomposed by moist heat. This stability can also be expected to be exhibited by phosphate covered titanium implants. The titanium/phosphate ion exchange material can be re-conditioned and then used for many other purposes. Zirconium phosphate complexes which are chemically closely related to titanium/phosphate compounds, exhibit similar properties and are used for the same purposes (Clearfield: Inorganic ion exchange materials. CRC press 1982, Boca Raton FI. USA).

The method described below is designed to be the last step in the manufacture process of titanium (or zirconium) implants. It is important that this manufacturing is performed by a process which reduces the oxidation of the machined titanium implant to a minimum. This facilitates the subsequent removal of oxide, as discussed below. Titanium implants or implants of certain titanium alloys are suitable for this method. As metallic titanium is always covered by an inert layer of oxide, an initial step is then to displace the oxide layer mechanically. Grit blasting with diamond powder or other suitable abrasives or methods is a possible approach. The procedure should be performed in an oxygen-free environment (preferably under argon). After mechanical removal of oxygen, the titanium metal is transferred to a phosphate solution where the titanium receives a layer of complex bonded phosphate which is selectively taken up due to the polydentate nature of the phosphate.

Chemically the oxide layer of titanium can be removed partially or completely, by the use of halogen acids. The halogens represent ligands with affinity for the complex forming transition metal titanium in the absence of oxygen-containing ligands, which have a higher affinity. Dilute hydrofluoric acid is known to react with titanium atoms to form a complex anion: (TiF₆)³⁻.

Treatment with HCl can be performed at temperatures between 20 and 80°C and should last for from 10 min to 24 hours or more.

Suitable concentrations of hydrofluoric acids are from 0.1 to 0.5% w/vol with a pH of 2.5-3.0, and treatment should last for a period of up to 2 minutes, generally from 30 to 60 seconds, depending on the thickness of the oxide layer. If a thick layer of oxide covers the implant the treatment can be performed at temperatures up to 60°C. A preferred treatment is at up to 50°C for up to 30 seconds.

These procedures will cause a displacement of the oxygen of titanium oxide with fluorine when hydrofluoric acids are used. This reaction takes place at pH 2.5-3, which is the natural pH of highly diluted aqueous solutions of hydrofluoric acid. The corresponding pH of HCl is from pH from 1.0 -1.2. The next step in the chemical procedure involves transfer of the titanium implant directly to an aqueous solution of phosphate or a phosphate buffer, and is similar to the treatment of implants where the oxide is removed by mechanical means. As titanium is a 3d transition metal with a higher affinity for oxygen than for halogens, the transfer to the phosphate solution will involve replacement of the fluorine on the titanium surface with un-protonated phosphate groups. A phosphate group may contain from 2-4 oxygen moieties and is polydentate, and may therefore react selectively with the titanium surface as discussed above. Other methods can be used for removal of the oxygen layer, for example the use of strong phosphoric acid, or procedures involving use of ultrasound.

A special characteristic of the present invention is that the chemical property of the exposed metallic titanium surface is exploited to obtain complex bonded phosphate groups on the surface of titanium implants, a process which is impossible in oxide coated implants. Thus, the invention provides a titanium implant having increased retention after insertion in bone. The implant comprises phosphate groups bound to the metallic titanium, i.e. not only to titanium oxide.

The resulting titanium/phosphate complexes will interact with calcium in the tissue fluid when the implant is placed in bone, and thus, a firmly bonded bone is formed on the implant. This mechanism is identical for both mechanically or chemically treated titanium implants. The mechanism represents an example of direct bonding between implant metal and bone, as discussed previously. It is essential not to expose the fluorine treated implant to water rinses before it is placed in phosphate solutions, as this would tend to enhance oxide formation on the implant.

The aqueous solutions of phosphate should preferably contain at least 0.1mol/l of phosphate with a pH between 2 and 8, preferably between 6 and 8, in particular about pH 7.2. The phosphate, which may be inorganic or organic in the form of mono-, di- or polyphosphate, should only contain a minimum of calcium, and should preferably be in the form of solutions with a concentration from 0.01 to 1.0% w/v. Molecules with exposed carboxylic groups can also be used, for example EDTA. Organic phosphate can also be used including DNA, RNA, ATP, ADP and AMP. A suitable phosphate buffer will normally have a concentration of from 0.01 to 0.2 mole/litre. Teichoic acid, and phosphoproteins like casein, may be used as well as molecules carrying biological entities which may promote integration of the implants in bone.

The treatment in phosphate should last for from 1 min to 24 hours, preferably from 1 to 2 hours.

As mentioned above titanium phosphate is used for industrial scale ionic exchange operations such as the removal of heavy metals from drinking water. The titanium is here in the form of a "sponge" with a large surface area (Clearfield 1982). Titanium/phosphate complexes are known to be very stable, and may be stored in a 10 mmol/l sterile phosphate solution, or stored dry. This is opposed to fluorine-treated titanium implants which are labile and known to be hygroscopic. This issue is discussed later, see experiments with titanium tetrafluoride. The surface may thus decompose during storage due to exposure to oxygen or light.

The titanium implant according to the invention forms a firm attachment to bone following implantation and differs from prior art materials, in particular that described in WO 95/17217 and US 2005/0161440 (Ellingsen et al.). Another way of describing the characteristic surface of the material produced by Ellingsen et al. is that this surface acquires a layer of visible precipitate, when it is exposed to a saturated solution of calcium phosphate at pH 7.2 in a Hepes buffer. This is opposed to the effect on untreated implant surfaces which do not exhibit such a layer. The authors seem to assume that calcium accounts for the precipitation. This is obvious from claims 41-54 and 59-64. The authors also state that a layer of oxide should be present during the treatment of the titanium with calcium, according to claims 27-40. It is known that the oxide layer which usually covers titanium implants exhibits certain ion exchange properties, of which binding of cations is one aspect (Clearfield 1982). It appears that the authors may have related the observed calcium binding of titanium to such a mechanism. As a method to describe the characteristics of fluorine-treated titanium implants the method of Ellingsen et al. has certain merits. However, the use of Hepes buffer and the visible precipitation of material on the implants represent a serious problem, if this method is considered as an alternative treatment of implants which are produced for clinical purposes. Hepes buffers cause faster blood clotting (Robertson et al. Throm. Heamost. 35: 202, 1976) which makes it unsuitable for clinical use, and visible precipitation of material on the implant surface makes threaded implants, or closely fitting implants, useless.

In contrast to Ellingsen et al. the present invention involves a two step procedure: 1) removal of oxide from the titanium implant, mechanically by grit blasting with suitable abrasive particles, or chemically by use of halogen acids; and 2) the titanium being a 3d transition metal is thus exposed. The titanium metal is able to form complexes with specific ligands. Oxygen-containing ligands like OH- and H₂O or non-protonated phosphate groups are strong ligands whereas the halogens in comparison are weak. This is exploited in the present invention as the fluoride-treated titanium is transferred to a phosphate solution where abundant amounts of oxygen-containing ligands are available, and these will replace the halogens on the implant surface.

The method described by Ellingsen et al. involves the use of titanium implants which exhibit fluorine or fluoride on the surface (claim 15) when it is introduced in the bone, and the clinical effect is related to this phenomenon. This is in contrast to the present invention where the fluoride is removed from the implant surface by competition with the oxygen moiety of the phosphate, and the phosphate is an essential aspect of the implant surface, whereas fluoride (or fluorine) has this function on the implant treated according to Ellingsen et al. In particular the mechanical method, by which oxide is removed from titanium implants as described in the present invention, is quite unrelated to the implant treatment described by Ellingsen et al. as it does not necessarily include any use of fluoride.

The present invention takes into account the properties of the 3d titanium transition metal and its ligands, whereas Ellingsen et al. obviously do not consider this possibility. This is illustrated by claims 34-40, which prescribe the presence of titanium oxide on the titanium surface and claims 41-64 which deal with calcium on the surface. Calcium is not a ligand of the transition metal titanium.

Ellingsen et al is solely concerned with the role of calcium, in the calcium phosphate precipitation on implants. The role of phosphate is never discussed. The present invention involves complex bonded phosphate on the titanium implants in an essential aspect. Even treatment with, and deposition of, calcium phosphate on implant surfaces, is different from the present invention, where the reaction between phosphate and calcium takes place in bone, after implantation, and not before the implantation, as described by Ellingsen et al.

Furthermore, the mechanical method of oxide elimination described in the present invention, which does not prescribe use of fluoride treatment of the implant, is unrelated to the speculations made in Documentation on Astra Tech implants, Issue 3, 2005.

Although the invention has been described herein with particular reference to titanium and titanium alloy implants, it extends also to implants comprising zirconium and zirconium alloys. Such implants can be made by methods analogous to those described above.

The invention is further illustrated by way of the following Experiments and nonlimiting Examples and with reference to the accompanying Figures:
Fig. 1: SEM image showing enamel specimens covered with grey particles and of scattered larger and denser particles referred to as B and A, respectively.
Figs. 2 and 3: SEM images showing the loss of grey particles, whereas the white dense particles A remain.
Fig. 4: Schematic representation of the results from the animal experiments, showing the interfacial tensile force when the samples B have been pretreated compared with control implants A.

### Experiments with titanium tetrafluoride

The aim of this experiment was to examine the reaction products of titanium tetrafluoride exposed to water, and the interaction of these products with an enamel surface, which consists mainly of hydroxyapatite that is also closely related to bone and exhibits numerous phosphate groups on its surface; Ca₁₀(PO₄)₆(OH)₂. The aim was furthermore to examine whether titanium metal ions would form complexes with the phosphate groups on the enamel surface. Such a reaction would indicate that also the opposite is true: that titanium metal implants would react with soluble phosphate ions, as illustrated schematically:

-(phosphate) O-Ti

in the present experiment. This may be compared to -Ti-O (phosphate) which occurs when metallic titanium is exposed to a phosphate solution, as described. Thus, the mechanism is the same in both cases.

The enamel surface was furthermore thought to facilitate the identification of the reaction products such as calcium fluoride, which is known to form on enamel exposed to titanium tetrafluoride (Büyükyilmaz et al., Europ. J. Oral Sci. 5: 473, 1997).

The titanium tetrafluoride was obtained from a commercial source and prepared as a 4% w/vol aqueous solution. After incubation for 10 min, four enamel pieces where introduced into the solution. After another two minutes the solution was discarded, and the enamel specimens rinsed in de-ionized water and dried. One specimen was treated with 50 ml of KOH (1 mole/I) for 24 hours and then rinsed in distilled water and dried. The enamel specimens were then prepared for SEM and EDAX. The experiment showed clearly that titanium fluoride is unstable in water and that the phosphate titanium complex is stable in 1 mol/l KOH.

### Results and discussion

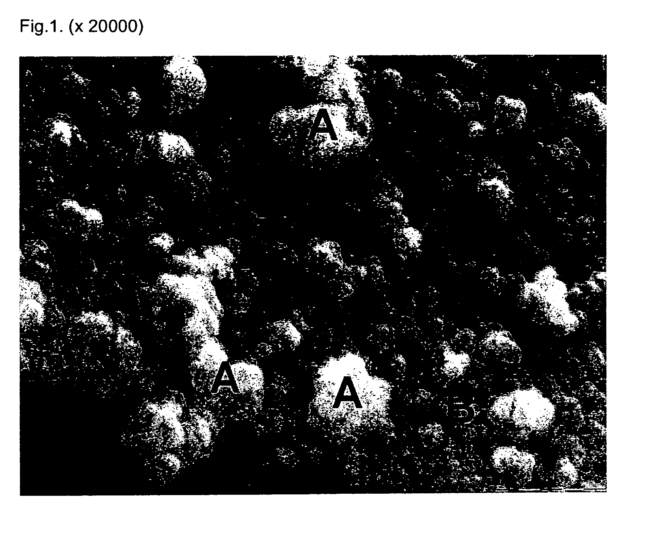

The pH of the freshly prepared solution of titanium tetrafluoride immediately dropped to pH 1.5. The SEM (Fig. 1) showed that the enamel specimens were covered with grey particles and with scattered larger and denser (white) particles referred to as B and A, respectively. The EDAX showed presence of Ca, P and Ti.

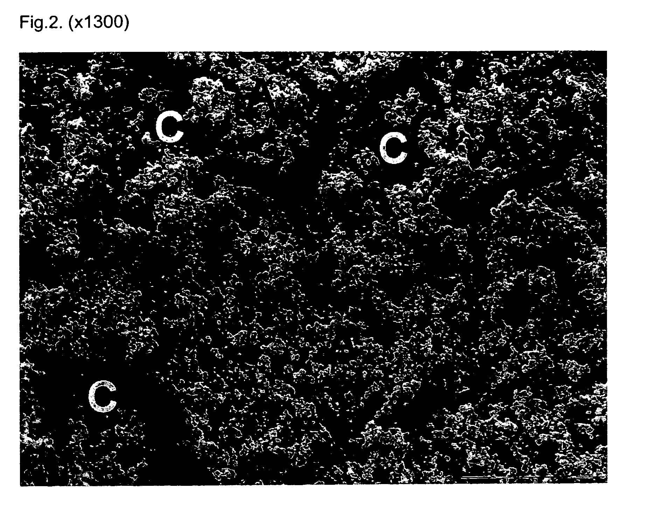

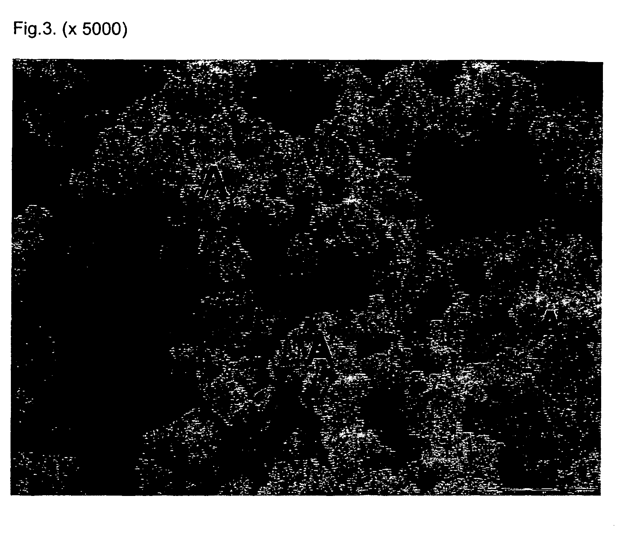

After KOH treatment, which is known to dissolve calcium fluoride, the SEM (Figs. 2 and 3) showed loss of the grey particles, whereas the white, dense particles (A) remained. (C) indicates naked areas of exposed enamel, which was previously covered with calcium fluoride. It could therefore be concluded that the grey particles (Fig. 1 B), which were lost after KOH treatment, were calcium fluoride. The white particles (Fig. 2A and 3A) consisted of titanium metal. It can be seen that the white dense particles were adsorbed to the enamel, probably by complex bonding to the phosphate groups exposed on the surface of the enamel.

The reactions can be interpreted as follows: the titanium tetrafluoride dissociates when exposed to water, and Ti causes hydrolysis (Ti + H₂O → TiOH + H⁺) and pH drops (Busalev et al., Inorgan. Chem. 17: 418, 1983). The fluoride forms an ionic HF at this low pH, which is known to react with the calcium on the surface of the enamel, and form calcium fluoride. This is a well known reaction. The white particles consist of titanium which is bonded to enamel through the oxygen moieties of the phosphate groups. This is presumably a complex binding between the ligand oxygen, and the transition metal titanium (Tveit et al., Caries Res. 17: 412, 1983). Thus, it was shown that titanium can form complexes with phosphate even at this low pH, where the oxygen moieties of the phosphate groups are protonated. It appears that the -phosphate-titanium complex was deposited prior to calcium fluoride, as this phase was visible mainly after the removal of calcium fluoride with KOH, as described above. It is thus clear that -phosphate-titanium complexes (as well as such complexes of -titanium-phosphate) can be formed without pretreatment with acids.

### Animal experiments

The aim of this experiment was to compare the retention in rabbit bone of untreated, coin-shaped titanium implants, with such implants treated with fluorine and phosphate, according to the present invention. New Zealand white rabbits were used and the experimental procedure was as described by Rönold and Ellingsen (Biomaterials 23: 21, 2002).

The rabbits had a weight of 4.6-5.4 Kg at the start of the study. The animals were kept in cages during the experimental period. Each animal was sedated by injection of a combination of fluanozonium 1.0 mg/kg and fentatylium 0.02 mg/kg (Hypnorm, Jannsen Pharmaceutical, Belgium), and locally anesthetized by xylocaine/adrenaline (AB Astra).

Two flat sites were drilled on each ulna of the rabbits, with a custom made drill which allows firm placement of two coin shaped implants. These had a diameter of 6.25 mm and a height of 1.95 mm, consisting of grade 2 titanium. The control implants (A), were blasted with TiO₂ particles of sizes from 180-220 µm, whereas the experimental implants (B) were identical, except that they in addition, had been treated with an aqueous solution of 0.2% hydrofluoric acid for 30 sec, followed by a 60 min treatment in a 0.1 M phosphate buffer pH 7.2. No water rinses were performed before the phosphate treatment.

The implants were removed after 8 weeks and tensile tests were performed with a Lloyds LRX machine, fitted with a calibrated load cell of 100N. The recorded force gives a direct assessment of the strength of the connection between implant and bone.

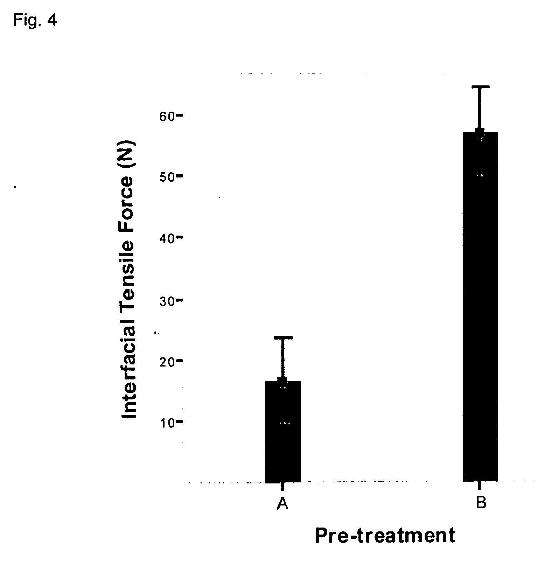

The results are given in Fig 4. It can be seen that the experimental implants had a significantly stronger affinity for bone surfaces than the untreated control implants. Furthermore, the tensile forces recorded for the control specimens varied markedly more than the experimental implants.

### Example 1

A titanium implant consisting of pure titanium and produced under argon was grit blasted with diamond powder under argon, until the oxide layer was eliminated. The implant was then transferred to a sterile, 0.1 mol/l phosphate buffer, for 4 hours or longer, and subsequently autoclaved and stored dry under sterile conditions.

If cooling is needed during clinical procedures, a sterile 10 mmol/l phosphate buffer, pH 7.2 should be provided for this purpose.

### Example 2

A titanium implant consisting of an alloy used for clinical purposes, and produced by conventional methods, was treated with a 0.2% aqueous solution of hydrofluoric acid for 30 sec, and then transferred directly to a 0.1 M phosphate buffer pH 7.0, containing 0.1% of sodium pyrophosphate, for 4 hours or longer. The implant was then sterilized by irradiation and stored dry in an oxygen free and dry atmosphere.

### Example 3

A titanium implant was produced under conventional conditions. The implant was grit blasted with oxide-free titanium powder and then with diamond powder under argon. The implant was then transferred to a saturated solution of casein for 2 hours, rinsed in a phosphate buffer and then irradiated and stored in a dilute phosphate buffer pH 7.

### Example 4

A titanium implant was produced under conventional conditions and then grit-blasted with 180-220 µm titanium particles. After this treatment the implant was grit blasted with diamond powder under argon, and transferred to a phosphate solution of pH 4 for 1 hour, and then to a phosphate buffer pH 7.2 for 1 hour. The implant was then autoclaved and transferred to a container with a dilute phosphate buffer pH 7.0 which covered the implant completely.

### Example 5

A zirconium implant was produced under argon and briefly grit blasted under argon. The implant was then transferred to a sterile 0.1 mol/I phosphate buffer pH 7.2 for 4 hours. The implant was then autoclaved and stored dry under sterile conditions.

## Claims

1. A titanium or titanium alloy implant which comprises phosphate groups complex bound to the surface thereof.

2. An implant as claimed in claim 1, wherein said phosphate groups are bound directly to the titanium surface of the implant by coordinate bonds.

3. An implant as claimed in claim 1 or claim 2, wherein the surface of the implant is substantially free from titanium oxide.

4. A process for preparing a titanium or titanium alloy implant, said process comprising:
(a) providing a titanium or titanium alloy implant;
(b) removing the oxide layer formed on the surface of the implant to expose the metallic titanium; and
(c) treating the exposed implant with a phosphate solution in the absence of oxygen.

5. The process of claim 4, wherein the removal of the oxide layer is accomplished by mechanical or chemical means under anaerobic conditions.

6. The process of claim 4 or claim 5 wherein the removal of oxide is carried out mechanically by grit blasting with diamond abrasives under argon.

7. The process of any one of claims 4 to 6, herein the removal of oxide is carried out by a soft rotating instrument under argon using diamond powder or any other abrasive which does not cause deposition of oxide or any foreign material on the implant surface.

8. The process of claim 4 or claim 5, wherein the removal of oxide is carried out with a halogen acid.

9. The process of claim 8, wherein the halogen acid is aqueous hydrofluoric acid (HF).

10. The process of claim 9, wherein the hydrofluoric acid has a pH in the range of from 2.5 to 3.0.

11. The process of any one of claims 8 to 10, carried out at room temperature for a period of up to 2 minutes.

12. The process of claim 11, carried out at up to 50°C for up to 30 seconds.

13. A titanium or titanium alloy implant obtainable by a process as claimed in any one of claims 4 to 12.

14. Use of titanium or a titanium alloy which comprises phosphate groups complex bound to the surface thereof in the manufacture of a medicament for use as an implant material, e.g. as a bone replacement material.

15. An implant or process for preparing an implant as defined in any one of claims 1 to 13 wherein said titanium or titanium alloy is replaced by zirconium or an alloy thereof.
